(19) Europäisches Patentamt
European Patent Office
Office européen des brevets

(11) Veröffentlichungsnummer : **0 403 841 B1**

(12) **EUROPÄISCHE PATENTSCHRIFT**

(45) Veröffentlichungstag der Patentschrift : **04.08.93 Patentblatt 93/31**

(51) Int. Cl.$^5$ : **C07C 43/303,** C07C 41/50

(21) Anmeldenummer : **90110243.4**

(22) Anmeldetag : **30.05.90**

(54) **Verfahren zur Herstellung von Arylacetaldehyddialkylacetalen.**

(30) Priorität : **19.06.89 DE 3919890**

(43) Veröffentlichungstag der Anmeldung : **27.12.90 Patentblatt 90/52**

(45) Bekanntmachung des Hinweises auf die Patenterteilung : **04.08.93 Patentblatt 93/31**

(84) Benannte Vertragsstaaten : **CH DE FR GB LI NL**

(56) Entgegenhaltungen :
**AT-B- 174 372**
**DE-A- 2 913 677**

(56) Entgegenhaltungen :
**DE-B- 1 077 650**
**DE-C- 821 203**
**FR-A- 1 327 650**
**FR-A- 2 577 920**

(73) Patentinhaber : **HÜLS AKTIENGESELLSCHAFT**
**Paul-Baumann-Strasse 1**
**W-4370 Marl 1 (DE)**

(72) Erfinder : **Theis, Christoph, Dr.**
**Ewaldstrasse 33**
**W-5216 Niederkassel 6 (DE)**
Erfinder : **Latz, Wilfried**
**Wahner Strasse 40b**
**W-5216 Niederkassel 3 (DE)**

**EP 0 403 841 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft ein Verfahren zur Herstellung von Arylacetaldehyd-dialkylacetalen durch Umsetzung von ein- oder mehrfach durch Halogenmethyl-gruppen substituierte Aromaten oder Heteroaromaten - die gegebenenfalls am aromatischen Kern eine oder mehrere unter den Synthesebedingungen inerten Gruppen tragen - mit Kohlenmonoxid, Wasserstoff, Orthoestern und Alkoholen in Gegenwart eines Halogenwasserstoff-Akzeptors und einem Metall-Carbonyl-Katalysator bei erhöhtem Druck und erhöhter Temperatur. Die Herstellung von Arylacetaldehydacetalen, besonders der Dimethyl- und Diethylacetale, ist nach dem Stand der Technik aufwendig und schwierig.

Die Synthese von Phenylacetaldehyd-dimethyl- bzw. -diethylacetal kann z.B. durch Umsetzung der Grignard-Verbindung von Benzylchlorid mit Triethylorthoformiat (GB-PS 823 958), durch Oxidation von Cyclooctatetraen mit Persäuren/Methanol (US-PS 2 856 431), durch säurekatalytische Umlagerung eines 1,2-Dimethoxycyclooctatetraens (DE-AS 1 077 650) oder durch oxidative Alkohol-Addition an Styrol in Gegenwart von Edelmetallkatalysatoren/Alkylnitrilen (EP-OS 055 108) erfolgen. Andere Verfahren beschreiben die Darstellung durch Umlagerung von Styroloxiden in Gegenwart von Katalysatoren (EP-PS 153 692; BE-PS 811 382) mit nachfolgender Acetalisierung des gebildeten Aldehyds. Nur wenige Verfahren zur Herstellung von substituierten Arylacetaldehydacetalen sind bekannt. In mäßiger Ausbeute wird p-Fluorphenylacetaldehyd-diethylacetal aus der Grignard-Verbindung des p-Fluorbenzylchlorids und Triethylorthoformiats gewonnen (FR-PS 1 327 160).

Arylacetaldehyd-acetale werden nach der FR-PS 2 577 920 aus Halogenmethyl-substituierten Aromaten mit Kohlenmonoxid und Wasserstoff in Gegenwart eines basischen Mittels, von Alkoholen und eines Cobalt-Carbonyl-Katalysators erhalten. Maßgebend für das Gelingen der Reaktion ist die Gegenwart eines inerten organischen Co-Solvens, wie Toluol, das die Einhaltung einer bestimmten Dielektrizitäts-Konstante gewährleistet. Dabei werden jedoch hohe Anteile eines nicht destillierbaren Rückstands gebildet. Neben den Acetalen entstehen größere Mengen von weiteren Stoffen, besonders auch die nicht gewünschten Arylessigsäureester. Die Reaktion wird mit steigenden Mengen schwierig ausführbar und ergibt schon im 1 Mol-Maßstab verminderte Ausbeuten und geringere Spezifität.

Es bestand daher die Aufgabe, Arylacetaldehyd-acetale in einfacher Weise, wirtschaftlich und mit möglichst hoher Reinheit und Ausbeute zu gewinnen.

Diese Aufgabe wird erfindungsgemäß durch katalytische Umsetzung der Halogenmethylaromaten mit CO, $H_2$ und Orthoestern und in Gegenwart einer basischen Verbindung in mindestens äquivalenten Mengen, bezogen auf die Halogenmethylaromaten, gelöst. Die Orthoester dienen dabei als wasserbindende Mittel. Als Halogenmethylaromaten sind die Chlormethylaromaten und die Brommethylaromaten bevorzugt.

Gegenstand der Erfindung ist das Verfahren zur Herstellung von Arylacetaldehyd-dialkylacetalen mit einer bzw. mehreren Gruppen $-CH_2-CH(OR^2)_2$ und gegebenenfalls einem bzw. mehreren bei der Reaktion inerten Substituenten $R^1$ der aromatischen Ringe durch Reaktion der entsprechenden Halogenmethylaromaten mit einer und mehreren Gruppen $-CH_2Hal$ mit Hal = F, Cl, Br oder J und gegebenenfalls denselben inerten Substituenten $R^1$ in Gegenwart einer Metallcarbonylverbindung eines Metalls der 8. Nebengruppe des Periodensystems als Katalysator mit mindestens äquivalenten Mengen von CO und $H_2$ bei erhöhtem Druck und erhöhter Temperatur in Gegenwart einer basischen Verbindung als Halogenwasserstoff-Akzeptor, dadurch gekennzeichnet, daß die Umsetzung in Gegenwart eines Orthoesters

$$R^3-C(OR^2)_3,$$

worin $R^2$ geradkettige oder verzweigte Alkylreste mit 1 bis 6 C-Atomen und $R^3$ Wasserstoff oder $R^2$ bedeuten, ausgeführt wird.

Besonders erfolgt die Umsetzung von Halogenmethyl-aromaten der Formeln

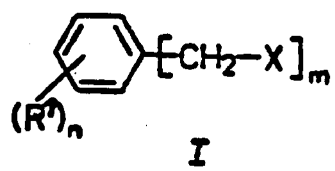

I

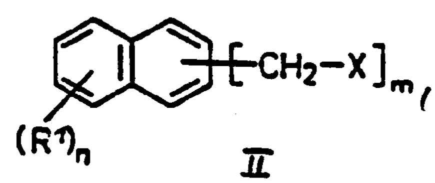

II

worin $R^1$, n, m und der Arylrest de genannte Bedeutung haben und X eines der Halogenatome F, Cl oder Br bedeuten.

Die Umsetzung der Chlormethylaromaten durch acetalisierende Hydroformylierung folgt bei Verwendung von Monochlormethylaromaten und Alkalikarbonat der Formel

$$2\ Ar{-}CH_2{-}Cl + 2\ CO + 2\ H_2 + 3\ R^3C(OR^2)_3 + M_2CO_3$$

$$\xrightarrow{Kat.} 2\ Ar{-}CH_2{-}CH(OR^2)_2 + 3\ R^3CO_2R^2 + 2\ R^2OH + CO_2 + 2\ MCl,$$

worin M = Na oder K bedeuten, $R^3$ Wasserstoff oder $R^2$ bedeuten, und $R^2$ geradkettige oder verzweigte Alkylreste und 1 bis 6 C-Atome sind.

Als Katalysatoren werden bevorzugt Carbonyle der Elemente Co, Ni oder Fe verwendet, wobei die Carbonyle des Cobalts bevorzugt werden. Sehr bevorzugt ist Dicobalt-octacarbonyl $Co_2(CO)_8$, welches als vorher hergestelltes Carbonyl eingesetzt wird oder aus Co-II-Verbindungen, besonders Co-II-Salzen, Co-II-Oxid oder -Hydroxid, basischem Co-Carbonat, Co-Salzen anorganischer und organischer Säuren, wie z.B. Co-Bromid, -Formiat, -Acetat, -Butyrat, -Naphthenat oder -Stearat während oder vor der Reaktion durch Umsetzung mit CO und $H_2$ gebildet wird.

Die Katalysatoren werden in Mengen von 0,25 bis 5 Mol-%, vorzugsweise 0,5 bis 2,0 Mol-%, berechnet als $Co_2(CO)_8$ bezogen auf den Halogenmethylaromaten, eingesetzt.

Unter den Bedingungen der obenstehenden Formel kann das Molverhältnis von CO zu $H_2$ im Bereich 5 : 1 bis 1 : 5 vorzugsweise im Bereich 2 : 1 bis 1 : 2 und sehr bevorzugt im Bereich 1 : 1 mit bis zu 20 % Toleranz liegen.

Der Gesamtdruck während der Umsetzung kann zwischen 5 und 30 MPa, vorzugsweise zwischen 5 und 25 MPa, sehr bevorzugt zwischen 10 und 25 MPa liegen. Die Reaktionstemperatur soll zwischen 90 und 130, vorzugsweise zwischen 95 und 110 °C liegen. In praxi wird das im Autoklaven befindliche und unter den vorgegebenen CO-/$H_2$-Drucken stehende heterogene Reaktionsgemisch so weit erwärmt, bis ein sichtbarer Druckabfall durch Verbrauch von CO und $H_2$ erfolgt. Die dann erreichte Reaktionstemperatur wird für die gesamte Umsetzung beibehalten.

Die einzusetzende Menge an basischen Mitteln beträgt mindestens äquivalente Mengen, vorzugsweise 1 bis 1,25 Äquivalente der entstehenden Menge von Chlorwasserstoff. Geeignete basische Mittel sind u.a. Carbonate, Hydrogencarbonate, Hydrogenphosphate, ggf. auch Hydroxide des Natriums oder Kaliums. Ein Überschuß der basischen Mittel ist nicht kritisch, jedoch werden bevorzugt stöchiometrische Mengen eingesetzt. Bevorzugt sind Alkali-Carbonate und -Hydrogencarbonate und von diesen das Natriumcarbonat und -hydrogencarbonat. Bei Verwendung von Alkalicarbonaten als Chlorwasserstoff-Akzeptor sind nach der Reaktionsgleichung 1,5 Mole Orthoester, bei Verwendung von Hydrogencarbonaten 2,0 Mole Orthoester je Mol umgesetzte Chlormethylgruppe zu verwenden. Die Orthoester sind in mindestens äquivalenten Mengen gemäß der Reaktionsgleichung einzusetzen. Ein Überschuß von bis zu 50 Mol-% Orthoester ist möglich und verbessert die Ausbeute.

Die Verwendung von Orthoestern als acetalisierendes und wasserbindendes Mittel bei dieser Carbonylierungs-Reaktion ist neu und aus mehreren Gründen überraschend: Nach P. Piacenti und M. Bianchi: Organic Synthesis via Metal Carbonyls - Vol . II, S. 28 ff (Ed. : I. Wender und P. Pino, Verlag Willey & Sons, New York 1977) werden Orthoester bei der Einwirkung von CO und $H_2$ (1 : 1) in Gegenwart von Dicobaltoctacarbonyl und ggf. Alkoholen bei Drucken über 8 MPa und Temperaturen über 80 °C in hohen Ausbeuten rasch hydroformyliert und gleichzeitig acetalisiert. Bei den annähernd gleichen Bedingungen des vorliegenden Verfahrens unterbleibt überraschend diese Umsetzung des Orthoesters mit CO und $H_2$, es sei denn, hohe Überschüsse von Orthoester sind anwesend. Von den Orthoestern (Orthocarbonsäure-trialkylestern) in der Definition des Patentanspruchs sind die Orthoameisensäure-methyl- und -ethylester bevorzugt. Es war ferner nicht zu erwarten, die die Acidität des Reaktionsmediums in Anwesenheit der basischen Mittel ausreichen würde, eine praktisch quantitative Acetalisierung der in situ erzeugten Aldehyde zu gewährleisten. Andererseits wird überraschend die Acidität trotz der Heterogenität der Reaktionsmischung nicht so hoch, daß die Wirksamkeit des Katalysators beeinträchtigt ist. Der Zusatz geringer Mengen von Alkoholen verbessert die Ausbeute an Acetalen und erleichtert die Durchführung der Reaktion. Sehr bevorzugt wird derselbe Alkohol zugesetzt, der aus dem jeweils verwendeten Orthoester gebildet wird. Die Menge der Chlormethylaromaten zu Alkohol soll 5 : 1 bis 1 : 5 Mol betragen. Überraschend bilden sich nicht Arylessigsäureester, die aus Benzylchloriden in Abwesenheit von Orthoester entstehen.

Als Ausgangsstoffe dienen besonders Halogenmethylaromaten der Formel I und II, in denen die Summe m + n der Substituenten im Falle der Formel I maximal 6 und im Falle der Formel II maximal 8 ist. Bevorzugt sind Mono(halogenmethyl)-aromaten mit m = 1 und in zweiter Linie Di(halogenmethyl)-amomaten mit m = 2, in denen die Zahl der Substituenten $R^1$ vorzugsweise 1, 2 oder 3 beträgt. Die Zahl der Substituenten $R^1$ beträgt sehr bevorzugt 1. Als Arylreste sind die carbocyclischen Reste Phenyl, gegebenenfalls Naphthyl, jedoch auch Heteroaromaten wie Thienyl- (Thiophenyl-), Pyridyl-, Chinolinreste oder Indolreste möglich, sowie weitere ein-

und mehrringige substituierte oder unsubstituierte Arylreste. Bevorzugt sind Arylreste mit ein oder zwei nur aus Kohlenstoff bestehenden Ringen. In den Ausgangsstoffen entspricht der Arylrest und der Substituent $R^1$ den Verfahrensprodukten.

Das erfindungsgemäße Verfahren hat den Vorteil einer einfachen und sicheren Durchführung der Reaktion sowie einer hohen Spezifität, d.h. der geringen Bildung von Nebenprodukten und hoher Ausbeuten.

Die Aufarbeitung kann, ggf. nach Entfernung des Alkalihalogenats und Zusatz von Wasser, durch Zerstörung des Carbonyl-Katalysators mit Hilfe von Wasserstoffperoxid als wässrige Lösung bei einem pH-Wert zwischen 4 und 5 erfolgen. Die Suspension trennt sich in eine wässrige und eine organische Phase. Danach werden vorzugsweise die Leichtsieder d.h. die Stoffe mit niedrigen Siedepunkten als die Siedepunkte des Produktes entfernt. Nach Versetzen mit geringen Mengen Alkali hydroxid oder dessen wässriger Lösung wird durch nachfolgende Vakuum-Destillation und ggf. anschließender Rektifikation das Produkt gewonnen.

Beispiel 1

In einen 1-Ltr.-Hubautoklaven werden 189,8 g Benzylchlorid (1,5 mol), 318,0 g Orthoameisensäure-trimethyl-ester (3,0 mol), 32,0 g Methanol (1,0 mol), 80,0 g $Na_2CO_3$ (0,75 mol) und 7,5 g $Co_2(CO)_8$ (1,46 Mol-%, bez. auf Benzylchlorid) eingefüllt.
Nach Spülen mit CO wurden 10 MPa Wasserstoff und 10 MPa CO aufgepreßt und der Reaktor aufgeheizt. Bei einer Innentemperatur von 100 °C setzt die Reaktion ein, die verbrauchten Gase werden im Verhältnis von 1 : 1 im Druckintervall 22 bis 24 MPa entsprechend dem Fortschreiten der Reaktion ergänzt; nach 1,5 h ist die Gasaufnahme abgeschlossen, und der Reaktor wird nach einer Nachreaktionszeit von 1 h abgekühlt und entspannt.
Die dem Reaktor entnommene Suspension wird wie folgt aufgearbeitet: Nach dem Gaschromatogramm der Flüssigphase ist das eingesetzte Benzylchlorid praktisch quantitativ umgesetzt. Der Wassergehalt beträgt weniger als 0,25 %. Es wird mit 350 ml Wasser versetzt. Das entstehende 2-Phasen-System wird unter gutem Rühren bei einem pH-Wert von 4,5 mit 15 ml 30 Gew.-%igem wäßrigem Wasserstoffperoxid versetzt und nachfolgend 1 h refluxiert. Nach dem Abkühlen wird die organische Phase abgetrennt, mit 50 ml 5 Gew.%iger Natronlauge gewaschen und unter Normaldruck destilliert. Nach Abdestillieren der Leichtsieder werden bei einer Sumpf-Temperatur von 120°C 4,5 g 50 Gew.%ige Natronlauge zugesetzt, 10 Minuten bei dieser Temperatur gerührt und anschließend der abgekühlte Sumpf einer Vakuum-Destillation unterworfen.
Man erhält 206,9 g Destillat (Kp = 87 bis 91 °C bei 10hPa).
Es verbleiben 16,9 g Destillationsrückstand.
Das Gaschromatogramm dieses Roh-Produktes weist neben 3,2 Fl.-% 2-Phenyl -ethanol einen Gehalt an Phenylacetaldehyd-dimethylacetal von 95,4 Fl .-% aus; dies entspricht einer Acetal-Ausbeute von 79,3% bez. auf einges. Benzylchlorid. Das erhaltene Roh-Produkt wird durch Rektifikation zu einem Produkt einer Reinheit von über 99,5 % aufgearbeitet.

Vergleichsbeispiel (nach FR-PS 2 577 920 Beisp. 13)

In einen 1-Ltr.-Hubautoklaven werden 75,9 g Benzylchorid (0,6 mol), 227,4 g Ethanol (4,94 mol), 192 ml Toluol, 31,9 g Soda (0,3 mol) und 7,0 g $Co_2(CO)_8$ (3,4 Mol-% bez. auf Benzylchlorid) vorgelegt. Der Autoklav wird verschlossen, mit CO gespült und nach Aufpressen von 2,5 MPa Wasserstoff und 2,5 MPa CO auf eine Innentemperatur von 80 °C gebracht. Mit einsetzender Gasaufnahme werden CO und Wasserstoff im Verhältnis von 1 : 1 im Druckintervall 5 bis 6 MPa bis zur Beendigung der Gasaufnahme ergänzt.

Nach Abkühlen und Entspannen des Reaktors wird die Reaktions-Lösung wie in Beispiel 1 aufgearbeitet: Man erhält 52,3 g Roh-Produkt durch Destillation, als Rückstand verbleiben 34,8 g nicht destillierbare Substanzen. Die gaschromatographische Untersuchung des Roh-Produktes weist einen Gehalt von 5,2 Fl.-% Benzylchlorid; 0,4 Fl.-% Phenylacetaldehyd; 0,2 Fl.-% 2-Phenyl-ethanol, 12,0 Fl.-% Phenylessigsäureethylester sowie 78,8 Fl.-% Phenylacetaldehyd-diethylacetal auf.
Aufgrund dieser Analyse errechnet sich eine Ausbeute von 37,3 % d. Th. an Phenylacetaldehyd-diethylacetal. Der Umsatz von Benzylchlorid beträgt 94,8 %.

Beispiel 2

Entsprechend Beispiel 1, jedoch unter Verwendung von 1 ,5 mol Natriumhydrogencarbonat anstelle von Soda wird die Verfahrensweise von Beispiel 1 wiederholt.
Aus dem erhaltenen Roh-Produkt errechnet sich eine Ausbeute von 77,7 % d.Th. an Phenylacetaldehyd-dimethylacetal.

Beispiel 3

Entsprechend Beispiel 1, jedoch unter Einsatz von 0,75 mol Kaliumcarbonat anstatt Soda wird nach Umsetzung und Aufarbeitung entsprechend Beispiel 1 Phenylacetaldehyd-dimethylacetal mit einer Ausbeute von 60,2 % erhalten.

Beispiel 4

Unter Verwendung von 1,5 mol Kaliumhydrogencarbonat wird nach Reaktionsdurchführung und Aufarbeitung entsprechend Beispiel 1 eine Ausbeute von 74,9% d.Th. an Phenylacetaldehyd-dimethylacetal erhalten.

Die in der nachfolgenden Tabelle 1 aufgeführten Beispiele 5 bis 22 wurden entsprechend Beispiel 1 ausgeführt und aufgearbeitet.

**Tabelle 1:**

| Beispiel | Mol Orthoester | Mol Methanol | Base [Mol] | Reaktions-Druck [MPa] | CO : $H_2$ | Acetal-Ausbeute [%] |
|---|---|---|---|---|---|---|
| 5 | 2,81 | 1,0 | $Na_2CO_3$ [0,75] | 22 - 24 | 2 : 1 | 58,8 |
| 6 | 2,81 | 1,0 | $Na_2CO_3$ [0,75] | 22 - 24 | 1 : 2 | 71,3 |
| 7 | 2,81 | 1,0 | $NaHCO_3$ [1,5] | 22 - 24 | 2 : 1 | 56,0 |
| 8 | 2,81 | 1,0 | $NaHCO_3$ [1,5] | 22 - 24 | 1 : 2 | 61,4 |
| 9 | 2,81 | 1,0 | $Na_2CO_3$ [0,75] | 5 - 7 | 1 : 1 | 57,1 |
| 10 | 2,81 | 1,0 | $Na_2CO_3$ [0,75] | 9 - 11 | 1 : 1 | 74,0 |
| 11 | 2,81 | 1,0 | $Na_2CO_3$ [0,75] | 13 - 15 | 1 : 1 | 78,8 |
| 12 | 2,81 | 1,0 | $Na_2CO_3$ [0,75] | 16,5 - 18,5 | 1 : 1 | 79,7 |
| 13 | 2,81 | 1,0 | $Na_2CO_3$ [0,75] | 19 - 21 | 1 : 1 | 81,2 |
| 14 | 2,81 | 1,0 | $Na_2CO_3$ [0,75] | 23 - 25 | 1 : 1 | 79,5 |
| 15 | 2,81 | 0 | $Na_2CO_3$ [0,75] | 13 - 15 | 1 : 1 | 72,9 |
| 16 | 2,81 | 0,75 | $Na_2CO_3$ [0,75] | 13 - 15 | 1 : 1 | 77,4 |
| 17 | 2,81 | 1,125 | $Na_2CO_3$ [0,75] | 13 - 15 | 1 : 1 | 79,4 |
| 18 | 2,81 | 1,5 | $Na_2CO_3$ [0,75] | 13 - 15 | 1 : 1 | 76,8 |
| 19 | 2,0 | 1,0 | $Na_2CO_3$ [0,75] | 13 - 15 | 1 : 1 | 72,4 |
| 20 | 2,25 | 1,0 | $Na_2CO_3$ [0,75] | 13 - 15 | 1 : 1 | 78,5 |
| 21 | 2,40 | 1,125 | $Na_2CO_3$ [0,75] | 13 - 15 | 1 : 1 | 80,0 |
| 22 | 2,625 | 1,125 | $Na_2CO_3$ [0,75] | 13 - 15 | 1 : 1 | 76,8 |

Beispiel 23

Entsprechend Beispiel 1 werden 1,5 mol Benzylchlorid, 1,0 mol Ethanol, 2,81 mol Orthoameisensäure-triethylester und 0,75 mol Natriumcarbonat umgesetzt und wie dorf beschrieben aufgearbeitet.
Aus der Masse des resultierenden Rohproduktes vom Kp 94 bis 104 °C (10 hPa) und dessen gaschromatographisch ermittelten Zusammensetzung ergibt sich eine Ausbeute von 72,8 % d.Th. an Phenylacetaldehyddiethylacetal.

Beispiel 24

Entsprechend Beispiel 23 werden die dort genannten Stoffe umgesetzt, wobei jedoch die Soda durch 1,5 mol Natriumhydrogencarbonat ersetzt wird. Nach Aufarbeitung entsprechend Beispiel 1 resultiert ein Roh-Destillat, aus dessen Zusammensetzung sich eine Ausbeute an Phenylacetaldehyddiethylacetal von 63,3 % d.Th. ergibt.

Die in der nachfolgenden Tabelle 2 aufgeführten Beispiele 25 bis 38 werden entsprechend Beispiel 1 unter Einsatz von substituierten Clormethyl-Aromaten, stöchiometrischer Menge Soda sowie mit doppelter molarer Konzentration an Orthoameisensäure-trimethylester pro reaktive Chlormethyl-Gruppe ausgeführt; die weiteren reaktionsspezifischen Parameter sind aus dieser Tabelle ersichtlich. Umsätze und Wassergehalte entsprechen den vorangegangenen Beispielen.

Nach den Aufarbeitungen entsprechend Beispiel 1 ergeben sich für die sehr reinen Roh-Produkte die in der Tabelle 1 aufgeführten Ausbeuten (bezogen auf den eingesetzten Chlormethyl-Aromaten). Alle erhaltenen Produkte weisen entsprechend ihrer Konstitution korrekte Spektroskopische Daten auf.

Tabelle 2

| Beispiel | Edukt | [mol] | Mol Methanol | $Co_2(CO)_8$-Konz. [Mol-%] | Reaktions-Druck [MPa] | Arylacetaldehyd-dimethylacetal Siedepunkt [°C /hPa] | Ausbeute [%] |
|---|---|---|---|---|---|---|---|
| 25 | m-Methoxy-benzylchlorid | [0,64] | 0,48 | 1,46 | 19 - 21 | 125 - 31 / 10 | 72,7 |
| 26 | o-Methyl-benzylchlorid | [1,50] | 1,125 | 1,46 | 16,5 - 18,5 | 95 - 103 / 10 | 75,0 |
| 27 | m-Methyl-benzylchlorid | [1,50] | 1,05 | 1,46 | 16,5 - 18,5 | 96 - 102 / 10 | 76,3 |
| 28 | p-Methyl-benzylchlorid | [1,50] | 1,125 | 1,46 | 23 - 25 | 94 - 102 / 10 | 79,5 |
| 29 | p-Methyl-benzylchlorid | [1,50] | 1,125 | 0,50 | 16,5 - 18,5 | 94 - 102 / 10 | 74,1 |
| 30 | o-Chlor-benzylchlorid | [1,50] | 1,125 | 1,46 | 16,5 - 18,5 | 102 - 108 / 10 | 78,8 |
| 31 | m-chlor-benzylchlorid | [0,674] | 0,50 | 1,34 | 16,5 - 18,5 | 115 - 118 / 10 | 72,9 |
| 32 | p-Chlor-benzylchlorid | [1,0] | 4,00 | 1,46 | 16,5 - 18,5 | 116 - 120 / 10 | 48,4 |
| 33 | p-Chlor-benzylchlorid | [1,50] | 2,81 | 1,46 | 16,5 - 18,5 | 116 - 120 / 10 | 59,7 |
| 34 | p-Fluor-benzylchlorid | [1,00] | 2,50 | 1,46 | 16,5 - 18,5 | 89 - 94 / 10 | 74,1 |
| 35 | p-Fluor-benzylchlorid | [0,675] | 0,50 | 1,34 | 16,5 - 18,5 | 89 - 94 / 10 | 67,9 |
| 36 | m-Trifluormethyl-benzylchlorid | [0,51] | 0,38 | 1,49 | 20 | 79 - 87 / 10 | 65,8 |
| 37 | m-Methoxycarbonyl-benzylchlorid | [1,50] | 1,125 | 1,46 | 16,5 - 18,5 | 121 - 122 / 1,5 | 49,4 |
| 38 | 1-Chlormethyl-naphthalin | [1,50] | 1,125 | 1,46 | 16,5 - 18,5 | 139 - 142 / 4,0 | 51,0 |

Beispiel 39

Entsprechend Beispiel 1 werden 0,75 mol p-Chlormethylbenzylchlorid, 3,0 mol Orthoameisensäure-trimethylester, 1,125 mol Methanol, 0,75 mol $Na_2CO_3$ sowie 7,5 g $Co_2(CO)_8$ eingesetzt. Die Reaktion verläuft bei Temperaturen von 106 bis 8 °C bei einem Druck von 16,5 - 18,5 MPa.
Nach entsprechender Aufarbeitung erhält man ein Roh-Produkt von Kp = 126 bis 8 °C (1,0 hPa), aus dessen Zusammensetzung sich eine Ausbeute an 4-(2',2'-Dimethoxyethylphenyl)-acetaldehyddimethylacetal von 34,2 d.Th. errechnet.

Beispiel 40

In einem 2-Ltr.-Hubautoklaven werden 421,4 g (3,0 mol) p-Methyl-benzylchlorid, 596,3 g (5,625 mol) Orthoameisensäuretrimethylester, 72,0 g (2,25 mol) Methanol, 160,0 g (1,5 m) $Na_2CO_3$ und 10,3 g $Co_2(CO)_8$ (1,0 mol-% bez. auf einges. p-Methylbenzylchlorid) eingefüllt. Nach Spülen mit CO wird CO und $H_2$ im Verhältnis 1 : 1 aufgepreßt und die Umsetzung bei 102 °C und einem Reaktionsdruck von 16,5 bis 18,5 MPa durchgeführt. Die Aufarbeitung der Reaktions-Suspension erfolgt entsprechend Beispiel 1 unter Einsatz von 700 ml $H_2O$ und 20 ml 30Gew.-%igem $H_2O_2$. Die abgetrennte organische Phase wird gemäß Beispiel 1 unter Verwendung von 7,5 g 50 Gew.-%iger Natronlauge destillativ aufgearbeitet.
Bei der Vakuum-Destillation des Sumpfes werden 454,8 g Rohprodukt mit einem Acetal-Gehalt von 94 Fl.-% erhalten. Ausbeute: 79,2 % d.Th. an p-Methylphenyl-acetaldehyd-dimethylacetal.

Beispiel 41

Beispiel 40 wird wiederholt, jedoch unter Einsatz von 477,0 g (4,5 mol) Orthoameisensäure-trimethylester. Nach Aufarbeitung und Destillation gemäß Beispiel 40 wurden 439,3 g Roh-Produkt mit einem Acetal-Gehalt von 92,6 Fl.-% erhalten. Ausbeute: 75,4 % d.Th. p-Methylphenyl-acetaldehyd-dimethylacetal.

**Patentansprüche**

1. Verfahren zur Herstellung von Arylacetaldehyd- dialkylacetalen mit einer bzw. mehreren Gruppen $-CH_2-CH(OR^2)_2$ und gegebenenfalls einem bzw. mehreren bei der Reaktion inerten Substituenten $R^1$ der aromatischen Ringe durch Reaktion der entsprechenden Halogenmethylaromaten mit einer und mehreren Gruppen $-CH_2Hal$ mit Hal = F, Cl, Br oder J und gegebenenfalls denselben inerten Substituenten $R^1$ in Gegenwart einer Metallcarbonylverbindung eines Metalls der 8. Nebengruppe des Periodensystems als Katalysator
mit mindestens äquivalenten Mengen von CO und $H_2$ bei erhöhtem Druck und erhöhter Temperatur
in Gegenwart einer basischen Verbindung als Halogenwasserstoff-Akzeptor
dadurch gekennzeichnet, daß die Umsetzung in Gegenwart eines Orthoesters

$$R^3-C(OR^2)_3$$

worin $R^2$ geradkettige oder verzweigte Alkylreste mit 1 bis 6 C-Atomen und $R^3$ Wasserstoff oder $R^2$ bedeuten, erfolgt.

2. Verfahren nach Anspruch 1, dadurch gekennzeichnet, daß Arylacetaldehyd-dialkylacetale der Formeln

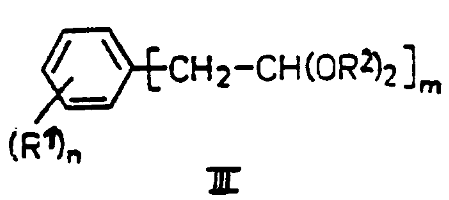

III

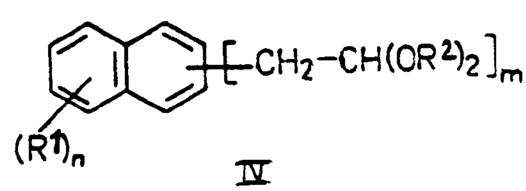

IV

worin im ggf. subsituierten Arylrest gegebenenfalls eines oder zwei der C-Atome durch ein Atom aus der Gruppe N, O oder S ersetzt sein kann und
worin der inerte Rest $R^1$ die Bedeutung H oder $R^2$; Halogen F, Cl, Br oder I , Alkoxy mit geradkettigen oder verzweigten Alkylresten mit 1 bis 6 C-Atomen; Perhalogenalkyl wie $CF_3$, $CCl_3$, $-CF_2-CF_3$, $-CCl_2-CCl_3$; Carboxy-methoxy bzw. Carboxy-
ethoxy; Hydroxy- oder Nitrogruppe hat und worin $R^2$ die Bedeutung geradkettige oder verzweigte Alkylreste mit 1 bis 6 C-Atomen besitzt und
worin n ganze Zahlen von 1 bis 5
und m ganze Zahlen von 1 bis 3 bedeutet, aus den strukturell jeweils entsprechenden Halogenmethylaromaten der Formeln

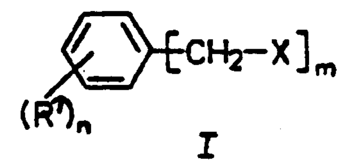

I

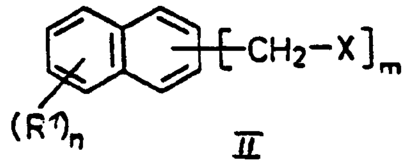

II

worin $R^1$, n, m und der Arylrest die genannte Bedeutung haben und X eines der Halogenatome F, Cl oder Br bedeuten,
hergestellt wird.

3. Verfahren nach einem der Ansprüche 1 oder 2, dadurch gekennzeichnet, daß Alkohole $R^2OH$ mit $R^2$ in genannter Bedeutung, vorzugsweise der aus dem Orthoester gebildete Alkohol, zugesetzt werden.

4. Verfahren nach einem der vorangehenden Ansprüche, dadurch gekennzeichnet, daß der Orthoester vorzugsweise der Orthoameisensäure-methyl- oder -ethylester ist.

5. Verfahren nach einem der vorangehenden Ansprüche, dadurch gekennzeichnet, daß die Aufarbeitung durch Zerstörung des Katalysators mit wässriger $H_2O_2$-Lösung, Abdestillation der Leichtsieder, Zugabe von Alkalilauge und Vakuumdestillation erfolgt.

## Claims

1. Process for the production of arylacetaldehyde-dialkylacetals with one or several groups $-CH_2-CH(OR^2)_2$ and possibly one or several substituents $R^1$ of the aromatic rings which are inert in the reaction, by reaction of the corresponding halomethyl aromatic compounds having one or several groups $-CH_2Hal$, with Hal = F, Cl, Br or I and possibly said inert substituents $R^1$, in the presence of a metal carbonyl compound of a metal of the 8th sub-group of the Periodic system as catalyst, with at least equivalent amounts of CO and $H_2$ at elevated pressure and elevated temperature in the presence of a basic compound as halogen chloride acceptor, characterised in that the reaction takes place in the presence of an orthoester

$$R^3-C(OR^2)_3$$

wherein $R^2$ denotes straight-chain or branched alkyl groups with 1 to 6 C-atoms and $R^3$ denotes hydrogen or $R^2$.

2. Process according to claim 1, characterised in that there is produced arylacetaldehyde-dialkylacetals of the formulae

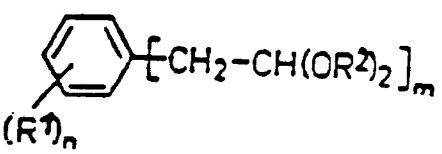

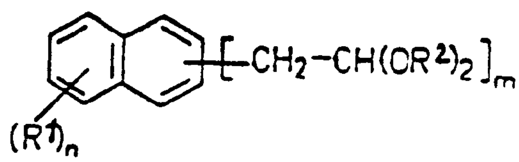

(III) (IV)

wherein, in the possibly substituted aryl group possibly one or two of the C-atoms can be replaced by an atom from the group N, O or S and wherein the inert group $R^1$ has the meaning H or $R^2$; halogen F, Cl, Br or I, alkoxy with straight-chain or branched chain alkyl groups with 1 to 6 C-atoms; perhaloalkyl like $CF_3$, $CCl_3$, $-CF_2-CF_3$, $-CCl_2-CCl_3$; carbomethoxy or carboethoxy; hydroxy or nitro group, and wherein $R^2$

possesses the meaning straight-chain or branched chain alkyl group with 1 to 6 C-atoms and wherein n denotes whole numbers of 1 to 5 and m denotes whole numbers of 1 to 3,
from the structurally respectively corresponding halomethyl aromatic compounds of the formulae

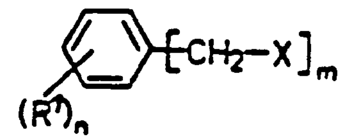

I

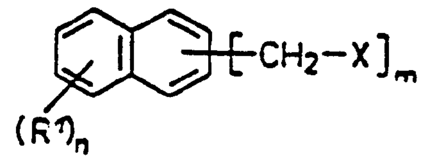

II

wherein $R^1$, n, m and the aryl group have the named meaning and X denotes one of the halogen atoms F, Cl or Br.

3. Process according to one of claims 1 or 2, characterised in that there are added alcohols $R^2OH$ with $R^2$ having the indicated meaning, preferably the alcohol formed from the orthoester.

4. Process according to one of the foregoing claims, characterised in that the orthoester is preferably the orthoformic acid methyl or ethyl ester.

5. Process according to one of the foregoing claims, characterised in that the working up takes place by destruction of the catalyst with aqueous $H_2O_2$ solution, distillation off of the low boiling substances, addition of alkali hydroxide and vacuum distillation.

## Revendications

1. Procédé pour préparer des acétals dialkyliques d'arylacétaldéhyde comportant un ou plusieurs groupes :

$$-CH_2-CH(OR^2)_2$$

et éventuellement un ou plusieurs substituants $R^1$, inertes dans la réaction, des noyaux aromatiques, par réaction des halogénométhylaromatiques correspondants, comportant un ou plusieurs groupes $-CH_2Hal$ avec Hal = F, Cl, Br ou I et éventuellement les mêmes substituants inertes $R^1$, en présence d'un composé métal-carbonyle, d'un métal du sous-groupe VIII du système ou tableau périodique , comme catalyseur, avec des quantités au moins équivalentes de CO et de $H_2$, sous pression élevée et à température élevée, en présence d'un composé basique comme accepteur d'hydracide halogéné,procédé caractérisé en ce que la réaction a lieu en présence d'un ortho-ester

$$R^3\text{- }C(OR^2)_3$$

dans lequel $R^2$ représente des restes alkyles linéaires ou ramifiés ayant 1 à 6 atomes de carbone et $R^3$ représente un atome d'hydrogène ou a le sens de $R^2$.

2. Procédé selon la revendication 1 , caractérisé en ce que les diacétals dialkyliques de formules

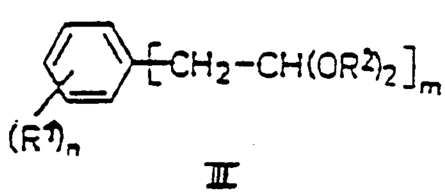

III

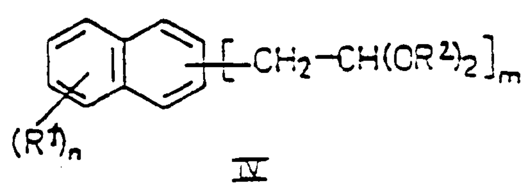

IV

(dans lesquelles éventuellement un ou deux des atomes de C du reste alkyle éventuellement substitué peut ou peuvent être remplacé(s) par un atome choisi dans l'ensemble formé par N,O ou S, et le reste inerte $R^1$ représente H ou a le sens de $R^2$, ou celui d'un halogène F, Cl, Br ou I, d'un groupe alcoxy comportant des restes alkyles linéaires ou ramifiés ayant 1 à 6 atomes de C; d'un groupe perhalogéno-alkyle comme $CF_3$, $CCl_3$, $-CF_2-CF_3$, $-CCl_2-CCl_3$; d'un groupe carboxy-méthoxy ou carboxy-éthoxy; d'un groupe

hydroxy ou nitro; $R_2$ représente des restes alkyles linéaires ou ramifiés ayant 1 à 6 atomes de carbone, et n représente des nombres entiers valant 1 à 5 et m représente des nombres entiers valant 1 à 3) sont préparés à partir d'hydrocarbures halogénométhyl-aromatiques de structure chaque fois correspondante , répondant aux formules:

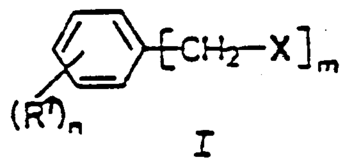

I

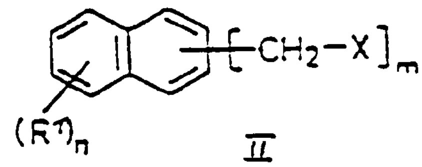

II

dans lesquelles $R^1$, n, m et le reste aryle ont le sens indiqué et X représente l'un des atomes d'halogène F, Cl ou Br.

3. Procédé selon l'une des revendications 1 ou 2, caractérisé en ce qu'on ajoute des alcools $R^2OH$, dans lesquels $R^2$ a le sens cité, avantageusement celui de l'alcool formé à partir de l'ortho-ester.

4. Procédé selon l'une des revendications précédentes, caractérisé en ce que l'ortho-ester est avantageusement l'ortho-ester méthylique ou éthylique de l'acide formique.

5. Procédé selon l'une des revendications précédentes, caractérisé en ce que le traitement de purification a lieu par destruction du catalyseur par une solution aqueuse de $H_2O_2$, enlèvement par distillation des substances à bas point d'ébullition, addition d'une lessive alcaline et distillation sous vide.